# FASCICULE DE BREVET EUROPEEN

(11) **EP 1 433 388 B1**
(45) Date de publication et mention de la délivrance du brevet: **15.08.2007**
(21) Numéro de dépôt: 03293210.5
(22) Date de dépôt: 18.12.2003
(51) Int. Cl.: A23L 1/337, A23L 1/30, A61K 36/02, A61P 19/10

(54) **Complément alimentaire à base d'algues**
Nahrungsmittelzusatz auf Basis von Algen
Algae-based food supplement

(30) Priorité: 23.12.2002 FR 0216556
(43) Date de publication de la demande: 30.06.2004
(73) Titulaire: Laboratoires Thalgo Nutrition, 83520 Roquebrune-Sur-Argens (FR)
(72) Inventeur: Brault, Dominique, 22740 Lezardrieux (FR); Lognone, Vincent, 22610 Lanmodez (FR); Menguy, Anne, 22500 Paimpol (FR); Sandoz, Robert, 06250 Mougins (FR); Sirop, Jean-Claude, 83110 Sanary-sur-mer (FR)
(74) Mandataire: Casalonga, Axel

(56) Documents cités:
- EP-A- 0 884 293
- WO-A-00/25602
- WO-A-92/11020
- BE-A- 626 884
- DE-A- 19 608 563
- FR-A- 1 485 766
- ASSOUMANI M.: "Aquamin , a natural calcium supplement derived from seaweed" AGRO FOOD INDUSTRY, vol. 9/10, 1997, pages 45-47, XP000864452

## Description

La présente invention concerne un complexe algal associant trois, éventuellement quatre algues marines, et son utilisation pour l'obtention d'un complément alimentaire, ainsi qu'en particulier un complément alimentaire, destiné à la femme ménopausée ou préménopausée, associant ledit complexe algal à des isoflavones de soja et, éventuellement, à de la vitamine D₃.

Les compléments alimentaires fournissant un apport de minéraux indispensables à la reminéralisation osseuse tels que le calcium, le magnésium, le bore et le zinc, sont connus et commercialisés depuis longtemps. Ces produits sont destinés en particulier aux personnes ayant un besoin accru en ces minéraux, telles que la femme enceinte ou qui allaite, et aux personnes présentant des risques de déminéralisation osseuse telles que les femmes ménopausées chez qui la carence en certaines hormones sexuelles se traduit par une moindre assimilation des sels minéraux et par une augmentation des risques d'ostéoporose.

Les compléments alimentaires fournissant un apport supplémentaire en calcium sont classiquement à base de carbonate de calcium insoluble d'origine minérale ou de coquilles d'huîtres broyées. Il est connu que la biodisponibilité de ces produits est assez médiocre, c'est-à-dire une grande partie du calcium est rejetée sans traverser la paroi intestinale et que seule une faible fraction du calcium absorbé dans le sang est réellement fixée et utilisée pour la reminéralisation osseuse.

Il existe un certain nombre de publications proposant des produits à base d'algues alimentaires riches en calcium et autres sels minéraux et un grand nombre de compléments alimentaires à base d'algues alimentaires sont actuellement commercialisés.

Une algue alimentaire particulièrement intéressante du point de vue de sa teneur en calcium et en magnésium est le *Lithothamnium calcareum* ou lithothamne (algue rouge encroûtante), commercialisé par exemple sous la dénomination AquaMin^{®} par la société Marigot. La poudre de *Lithothamnium calcareum* obtenue par micronisation de cette algue présente une porosité très élevée. Cette grande porosité améliore considérablement le taux d'absorption intestinale et donc la biodisponibilité des sels minéraux et notamment du calcium (voir en particulier les publications de Assoumani M.B. : AquaMin, a natural calcium supplement derived from seaweed. Agro-Food-Industry HI-Tech, sept/oct. 1997 : 45 - 47 et Physical-chemical properties of calcium sources, Agro-Food-Industry Hi-Tech, sept/oct, 1998 : 33 - 35.)

Le document DE 19608563 décrit un complément alimentaire à base d'algues contenant du Lithothamnium calcareum pulverisé et du Fucus vesiculosus micronisé.

La demanderesse a découvert qu'il était possible d'augmenter encore la biodisponibilité des sels minéraux du lithothamne en associant celui-ci à des proportions appropriées de deux autres algues brunes alimentaires de l'ordre des fucales, à savoir *Himanthalia elongata* et *Fucus vesiculosus,* et éventuellement à une troisième algue brune alimentaire qu'est *Ascophyllum nodosum.*

L'extrait d'*Himanthalia elongata* a de plus un effet synergique de potentialisation de la fixation du calcium.

La présente invention a donc pour objet un complexe algal constitué
- de 70 à 90 % en poids de *Lithothamnium calcareum* micronisé,
- de 7 à 13 % en poids d'extrait sec d*'Himanthalia elongata,* et
- de 6 à 17 % en poids d'extrait sec de *Fucus vesiculosus* ou de farine de *Fucus Vesiculosus* et d'*Ascophyllum nodosum.*

L'invention a également pour objet l'utilisation de ce complexe algal pour l'obtention d'un complément alimentaire.

On entend par "complément alimentaire" dans la présente invention des denrées alimentaires dont le but est de compléter le régime alimentaire normal et qui constituent une source concentrée de nutriments ou d'autres substances ayant, seuls ou en combinaison, un effet nutritionnel ou physiologique, commercialisés sous forme de doses unitaires telles que les gélules, les pastilles, les comprimés, les pilules, les sachets de poudre et autres formes similaires, permettant une absorption en unités mesurées de faible quantité.

Les proportions respectives des trois ou quatre ingrédients formant le complexe algal de la présente invention ont été choisies de manière à fournir un apport en nutriments équilibré, c'est-à-dire un apport permettant d'assurer aux être humains, en particulier à certaines périodes de leur vie (grossesse, allaitement, ménopause, andropause), un bon développement et un maintien dans un bon état de santé, et ce en des quantités correspondant à celles qui sont établies et recommandées à la lumière des données scientifiques généralement admises.

Le complexe algal selon l'invention présente en particulier une teneur en calcium comprise entre 215 et 280 mg par gramme de matière sèche, une teneur en magnésium comprise entre 14 et 18 mg par gramme de matière sèche, et une teneur en iode comprise entre 70 et 180 µg par gramme de matière sèche. Son originalité réside dans le fait que le calcium y est associé à de l'iode d'origine marine.

Les extraits secs d'*Himanthalia élongata* et de *Fucus vesiculosus* utilisés dans le complexe algal de la présente invention sont obtenus par extraction des algues séchées, à l'état broyé ou non, en suspension à 5 - 10 % dans de l'eau, à une température comprise entre la température ambiante et environ 60 °C, pendant une durée comprise entre environ 1 heure et 5 heures. L'extrait aqueux obtenu après élimination des parties insolubles selon des techniques connues, est concentré puis séché par atomisation. On peut éventuellement broyer la poudre ainsi obtenue jusqu'à une taille de particules souhaitée.

La demanderesse a découvert en outre qu'il était particulièrement intéressant d'utiliser le complexe algal selon l'invention dans un complément alimentaire, destiné à la femme ménopausée ou préménopausée, contenant, en plus du complexe algal, une certaine fraction de farine de soja.

En effet, la farine de soja contient, en tant qu'ingrédient essentiel, des isoflavones qui sont des phytoestrogènes, c'est-à-dire des molécules, synthétisées par des végétaux, ayant une structure proche de celle des oestrogènes.

L'administration d'isoflavones de soja est par conséquent typiquement envisagée lorsqu'un traitement hormonal substitutif classique constitué d'une association d'oestrogènes et d'une hormone progestative, n'est pas souhaitable chez certaines femmes en raison de fortes contre-indications ou d'effets secondaires gênants. Les isoflavones de soja permettent en effet d'atténuer un certain nombre d'inconvénients liés à la ménopause, tels que les bouffées de chaleur, sans pour autant présenter les effets secondaires propres aux oestrogènes.

Toutefois, certains thiocyanates antithyroïdiens présents dans le soja font que les régimes alimentaires riches en soja peuvent renforcer une hypothyroïdie qui apparaît souvent naturellement à partir de 50 ans. Dans ce contexte, l'apport en iode du complexe algal selon l'invention est particulièrement intéressant car il permet de pallier à un éventuel effet hypothyroïdien d'une administration de farine de soja.

La présente invention a par conséquent également pour objet un complément alimentaire, destiné à la femme ménopausée ou préménopausée, constitué
(a) de 50 à 90 % en poids, de préférence de 80 à 90 % en poids, de complexe algal selon l'invention, décrit ci-dessus,
(b) de 10 à 50 % en poids, de préférence de 10 à 20 % en poids, de poudre ou de farine de soja et, éventuellement,
(c) jusqu'à 10 % en poids d'un ou de plusieurs adjuvants ou additifs alimentaires,
la somme de (a), (b) et (c) étant égale à 100 %.

La teneur en farine de soja dépend, bien entendu, de sa teneur en isoflavones. Le complément alimentaire selon l'invention contient de préférence entre 2 % et 16 % en poids d'isoflavones de soja, par rapport à la somme de (a), (b) et (c).

Dans un mode de réalisation préféré du complément alimentaire selon l'invention, destiné à la femme ménopausée ou préménopausée, le complexe algal et la farine de soja sont associés à une concentration efficace de vitamine D₃. En effet, la vitamine D₃ est le principal facteur antirachitique et son utilisation dans le complément alimentaire de la présente invention permet de renforcer chez la femme ménaupausée ou préménopausée la fixation osseuse du calcium fourni. La concentration en vitamine D₃ est de préférence comprise entre 1 et 4 µg par gramme de complément alimentaire, de préférence entre 1,5 et 3 µg par gramme de complément alimentaire.

Comme indiqué ci-dessus, le complément alimentaire selon l'invention peut comprendre jusqu'à 10 % en poids d'un ou de plusieurs adjuvants ou additifs alimentaires. Ces adjuvants ou additifs alimentaires peuvent être choisis notamment parmi les colorants, les arômes, les agents conservateurs, les agents de délitement, les agents lubrifiants, les agents d'enrobage ou d'encapsulation.

Le complément alimentaire selon l'invention peut se présenter sous n'importe quelle forme compatible avec une absorption orale en une ou plusieurs prises journalières. Il peut se présenter notamment sous forme de gélules, de capsules, de comprimés, de pastilles ou de poudre libre, cette dernière étant conditionnée de préférence en sachets unitaires.

Le complément alimentaire à base d'algues et de farine de soja de la présente invention est conçu pour être consommé en une ou plusieurs prises alimentaires. Le poids unitaire des doses (gélules, comprimés, sachets de poudre, etc) est tel que l'absorption de deux à trois unités par jour permet l'administration des quantités souhaitées en nutriments.

Plus précisément, le complément alimentaire est conditionné en doses ayant un poids unitaire compris entre 200 mg et 2 g.

Les exemples de réalisation suivants illustrent l'invention.

### Exemple 1

### Complexe algal associant trois algues marines

| ingrédients | % en poids |
|---|---|
| *Lithothamnium calcareum* micronisé | 82,6 |
| Extrait sec d'*Himanthalia elongata* | 9,4 |
| Extrait sec de *Fucus vesiculosus* | 8,0 |

### Exemple 2

### Complexe algal associant quatre algues marines

| ingrédients | % en poids |
|---|---|
| *Lithothamnium calcareum* micronisé | 81,2 |
| Extrait sec d'*Himanthalia elongata* | 9,4 |
| Farine d'*Ascophyllum nodosum* | |
| et de *Fucus vesiculosus* | 9,4 |

### Exemple 3

### Complément alimentaire destiné à la femme ménopausée ou préménopausée, sous forme de gélule

| ingrédients | % en poids |
|---|---|
| Complexe algal | 84,96 |
| Farine de soja à 40 % d'isoflavones | 10,62 |
| Vitamine D₃ | 0,00029 |
| Silice hydratée | 1,70 |
| Silicate de magnésium | 1,70 |
| Stéarate de magnésium | 1,02 |

## Revendications

1. Complexe algal constitué
• de 70 à 90 % en poids de *Lithothamnium calcareum* micronisé
• de 7 à 13 % en poids d'extrait sec d*'Himanthalia elongata,* et
• de 6 à 17 % en poids d'extrait sec de *Fucus vesiculosus* ou de farine de *Fucus Vesiculosus* et d'*Ascophyllum nodosum.*

2. Complexe algal selon la revendication 1, **caractérisé par le fait qu'**il a une teneur en calcium comprise entre 215 et 280 mg par gramme de matière sèche, une teneur en magnésium comprise entre 14 et 18 mg par gramme de matière sèche, et une teneur en iode comprise entre 70 et 180 µg par gramme de matière sèche.

3. Utilisation du complexe algal selon la revendication 1 ou 2 pour l'obtention d'un complément alimentaire.

4. Complément alimentaire destiné à la femme ménopausée ou préménopausée, constitué
(a) de 50 à 90 % en poids de complexe algal selon la revendication 1 ou 2,
(b) de 10 à 50 % en poids de poudre ou de farine de soja et, éventuellement,
(c) jusqu'à 10 % en poids d'un ou de plusieurs adjuvants ou additifs alimentaires,
la somme de (a), (b) et (c) étant égale à 100 %.

5. Complément alimentaire selon la revendication 4, **caractérisé par le fait que** sa teneur en isoflavones de soja est comprise entre 2 % et 16 % en poids, rapportée à la somme de (a), (b) et (c).

6. Complément alimentaire selon la revendication 4 ou 5, **caractérisé par le fait qu'**il contient en outre de 1 à 4 µg/g, de préférence de 1,5 à 3 µg/g de vitamine D₃.

7. Complément alimentaire selon l'une des revendications 4 à 6, **caractérisé par le fait que** le ou les additifs ou adjuvants alimentaires éventuellement présents sont choisis parmi les colorants, les arômes, les agents conservateurs, les agents de délitement, les agents lubrifiants, les agents d'enrobage ou d'encapsulation.

8. Complément alimentaire selon l'une des revendications 4 à 7, **caractérisé par le fait qu'**il se présente sous la forme de gélules, de capsules, de comprimés, de pastilles ou de poudre libre.

9. Complément alimentaire selon l'une des revendications 4 à 8, **caractérisé par le fait qu'**il est conditionné en doses ayant un poids unitaire compris entre 200 mg et 2 g.

## Claims

1. Algae complex consisting
• of 70% to 90% by weight of micronized *Lithothamnium calcareum,*
• of 7% to 13% by weight of *Himanthalia elongata* dry extract, and
• of 6% to 17% by weight of *Fucus vesiculosus* dry extract or of *Fucus vesiculosus* and *Ascophyllum nodosum* meal.

2. Algae complex according to Claim 1, **characterized in that** it has a calcium content of between 215 and 280 mg per gram of solids, a magnesium content of between 14 and 18 mg per gram of solids, and an iodine content of between 70 and 180 µg per gram of solids.

3. Use of the algae complex according to Claim 1 or 2, for obtaining a food supplement.

4. Food supplement intended for menopausal or premenopausal women, consisting
(a) of 50% to 90% by weight of algae complex according to Claim 1 or 2,
(b) of 10% to 50% by weight of soybean powder or meal and, optionally,
(c) up to 10% by weight of one or more food adjuvants or additives,
the sum of (a), (b) and (c) being equal to 100%.

5. Food supplement according to Claim 4, **characterized in that** its soybean isoflavone content is between 2% and 16% by weight, relative to the sum of (a), (b) and (c).

6. Food supplement according to Claim 4 or 5, **characterized in that** it also contains from 1 to 4 µg/g, preferably from 1.5 to 3 µg/g, of vitamin D₃.

7. Food supplement according to one of Claims 4 to 6, **characterized in that** the food additive(s) or adjuvant(s) optionally present is (are) chosen from dyes, flavourings, preserving agents, disintegrating agents, lubricants, and coating or encapsulating agents.

8. Food supplement according to one of Claims 4 to 7, **characterized in that** it is in the form of gelatin capsules, capsules, tablets, lozenges or loose powder.

9. Food supplement according to one of Claims 4 to 8, **characterized in that** it is packaged in doses having a unit weight of between 200 mg and 2 g.

## Patentansprüche

1. Algenkomplex, bestehend aus
• 70 bis 90 Gew.-% *Lithothamnium calcareum,* mikronisiert,
• 7 bis 13 Gew.-% Trockenextrakt von *Himanthalia elongata,* und
• 6 bis 17 Gew.-% Trockenextrakt von *Fucus vesiculosus* oder Mehl aus *Fucus vesiculosus* und *Ascophyllum nodosum.*

2. Algenkomplex nach Anspruch 1, **dadurch gekennzeichnet, dass** er einen Calciumgehalt von 215 bis 280 mg pro Gramm Trockensubstanz, einen Magnesiumgehalt von 14 bis 18 mg pro Gramm Trockensubstanz und einen Iodgehalt von 70 bis 180 µg pro Gramm Trockensubstanz aufweist.

3. Verwendung des Algenkomplexes nach Anspruch 1 oder 2 für die Herstellung eines Nahrungsergänzungsmittels.

4. Nahrungsergänzungsmittel, das für Frauen in der Menopause oder vor der Menopause vorgesehen ist, bestehend aus
(a) 50 bis 90 Gew.-% Algenkomplex nach Anspruch 1 oder 2,
(b) 10 bis 50 Gew.-% Sojapulver oder Sojamehl, und gegebenenfalls
(c) bis zu 10 Gew.-% eines oder mehrerer Adjuvantien oder Zusatzstoffe für Lebensmittel,
wobei die Summe von (a), (b) und (c) 100 % ist.

5. Nahrungsergänzungsmittel nach Anspruch 4, **dadurch gekennzeichnet, dass** sein Gehalt an Isoflavonen aus Soja im Bereich von 2 bis 16 Gew.-%, bezogen auf die Summe von (a), (b) und (c), liegt.

6. Nahrungsergänzungsmittel nach Anspruch 4 oder 5, **dadurch gekennzeichnet, dass** es ferner 1 bis 4 µg/g und vorzugsweise 1,5 bis 3 µg/g Vitamin D₃ enthält.

7. Nahrungsergänzungsmittel nach einem der Ansprüche 4 bis 6, **dadurch gekennzeichnet, dass** der oder die Zusatzstoffe oder Adjuvantien für Lebensmittel, die gegebenenfalls enthalten sind, unter den Farbmitteln, Aromastoffen, Konservierungsmitteln, Hilfsmitteln für die Spaltung, Gleitmitteln, Stoffen zur Umhüllung oder Verkapselung ausgewählt sind.

8. Nahrungsergänzungsmittel nach einem der Ansprüche 4 bis 7, **dadurch gekennzeichnet, dass** es in Form von Gelatinekapseln, Kapseln, Tabletten, Pastillen oder losem Pulver vorliegt.

9. Nahrungsergänzungsmittel nach einem der Ansprüche 4 bis 8, **dadurch gekennzeichnet, dass** es in Einzeldosen von 200 mg bis 2 g konfektioniert ist.
